Europäisches Patentamt
European Patent Office
Office européen des brevets

(19)

(11) Veröffentlichungsnummer: **0 414 895 A1**

(12)

# EUROPÄISCHE PATENTANMELDUNG
## veröffentlicht nach Art. 158 Abs. 3 EPÜ

(21) Anmeldenummer: **89907370.4**

(22) Anmeldetag: **13.03.89**

(86) Internationale Anmeldenummer:
**PCT/SU89/00063**

(87) Internationale Veröffentlichungsnummer:
**WO 90/10625 (20.09.90 90/22)**

(51) Int. Cl.[5]: **C07D 239/557**, C07C 229/14, A61K 31/195

(43) Veröffentlichungstag der Anmeldung:
**06.03.91 Patentblatt 91/10**

(84) Benannte Vertragsstaaten:
**AT CH DE FR GB LI**

(71) Anmelder: **NAUCHNO-PROIZVODSTVENNOE OBIEDINENIE "BIOLAR"**
**Latviiskaya SSR**
**Olaine, 229014(SU)**

(72) Erfinder: **ZAMAKH, Vladimir Petrovich**
**ul. Mendeleeva, 24-87 Latviiskaya SSR**
**Olaine, 229014(SU)**
Erfinder: **BROD, Ivar Isakovich**
**ul. Lenina, 71-6**
**Riga, 226001(SU)**
Erfinder: **AUTSE, Alvis Albertovich**
**ul. Elgavas, 8-39 Latviiskaya SSR**
**Olaine, 229014(SU)**
Erfinder: **ARTJUKH, Maiga Alexandrovna**
**ul. Kirova, 8-8**
**Riga, 226010(SU)**
Erfinder: **BRITSE, Maiga Arnoldovna**
**ul. Tallinas, 84-10**
**Riga, 226009(SU)**
Erfinder: **FREIMANE, Zaiga Robertovna**
**ul. Lestenes, 6-2**
**Riga, 226002(SU)**
Erfinder: **NEUMYVAKIN, Ivan Pavlovich**
**proezd Shokalskogo, 6-148**
**Moscow, 129642(SU)**
Erfinder: **POLEVOI, Leonard Georgievich**
**ul. Mosfilmovskaya, 39-2-54**
**Moscow, 117330(SU)**
Erfinder: **ERGLIS, Andrei Paulovich**

**deceased(SU)**

(74) Vertreter: **von Füner, Alexander, Dr. et al**
**Patentanwälte v. Füner, Ebbinghaus, Finck**
**Mariahilfplatz 2 & 3**
**W-8000 München 90(DE)**

(54) **OROTAT DER -G(A)-AMINO--G(B)-PHENYL-BUTTERSÄURE.**

(57) γ -Amino- β -phenylbuttersäureorotat stellt eine Verbindung der

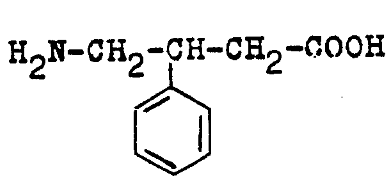 · 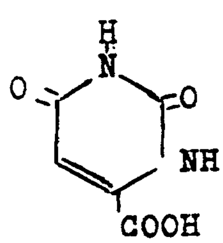

EP 0 414 895 A1

allgemeinen Formel dar.

Die neue Verbindung weist eine antihypoxische, sedative und antiarrhythmische Wirksamkeit auf und kann in der Medizin Verwendung finden.

# $\gamma$ -AMINO- $\beta$ PHENYLBUTTERSURE-OROTAT

## Anwendungsgebiet

Die vorliegende Erfindung bezieht sich auf die organische Chemie und betrifft insbesondere neue Verbindung - des $\gamma$ -Amino- $\beta$ -phenylbuttersäure-orotats. Die genannte Verbindung besitzt eine antihypoxische, sedative und antiarrhythmische Wirksameit.

## Zugrundeliegender Stand der Technik

Es sind verschiedene Verbindungen bekannt, die eine antihypoxische, sedative und antiarrhythmische Wirksamkeit aufweisen. So sind beispielsweise $\gamma$-Amino--$\beta$ -phenylaminosäurehydrochlorid (Phenibut) (M.D.Mashkovsky "Lekarstvennye sredstva" (Arzneimittel), I977, Moskau, Verlag "Meditsina", S. 87-88) und I-Karbamidomethylpyrrolidon-2 (Pyrazetam) (M.D.Mashkovsky, L.F. Boschina, A.I.Polezhaeva "Einige Besonderheiten der pharmakologischen Wirkung von Pyrazetam", Farmakologia i toksikologia (Pharmakologie und Toxikologie), I977, Nr. 6, S. 676-684) bekannt, die eine antihypoxische und sedative Wirksamkeit aufweisen.

Pyrazetam stellt eine pharmakologisch wirksame nootrope Verbindung dar, die einen Schutzeffekt bei verschiedenen Formen der Sauerstoffinsuffizienz besitzt, jedoch ist es nur bei hohen Dosen von 500 bis 2000 mg/kg effektiv.

Was $\gamma$ -Amino- $\beta$ -phenylbuttersäurehydrochlorid anbetrifft, so besitzt diese Verbindung eine antihypoksische und sedative Wirksamkeit, jedoch werden bei deren Anwendung die Hemmung der motorischen Aktivität, die Entwicklung der Gewöhnung des Organismus, die Reizung der Schleimhaut des Verdauungskanals nachgewiesen.

## Offenbarung der Erfindung

Die angemeldete Verbindung ist neu und in der Fachliteratur nicht beschrieben.

Der Erfindung liegt die Aufgabe zugrunde, eine neue Verbindung zu entwickeln, die eine antihypoxische, sedative und antiarrhythmische Wirksamkeit besitzt, keine Gewöhnung des Organismus bei der dauernden Medikation hervorruft.

Diese Aufgabe wird dadurch gelöst, dass die beanspruchte Verbindung - das $\gamma$ -Amino- $\beta$ -phenylbuttersäureorotat erfindungsgemäss folgende allgemeine Formel:

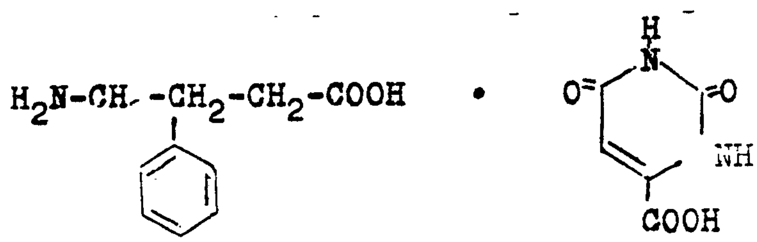

aufweist.

Die neue Verbindung stellt eine weisse kristalline Substanz von 268 bis 270 °C Schm.p. dar, die in wässrigen Alkalilösungen löslich, schwer wasserlöslich, bei der Aufbewahrung beständig ist.

Die Struktur der Verbindung wurde durch die Angaben der Elementaranalyse bekräftigt. Für $C_{15}H_{17}N_5O_6$:

Berechnet, %: C 53,73: N I2,53: H 5,06.
Gefunden, %: C 53,96: N I2,76: H 5,96
C 54,IO: N I2,94: H 5,42.

Beste Ausführungsform der Erfindung Es wurde die antihypoxische, sedative und antiarrhythmische Wirksamkeit der neuen Verbindung im Tierversuch untersucht.

Zur Bestimmung der sedativen Wirksamkeit wurde die motorische und Orientierungsaktivität und die motorische Fähigkeit in Versuchen an Mäusen und Ratten untersucht.

Den Versuchstieren wurden die beanspruchte Verbindung, $\gamma$ -Amino- $\beta$ -phenylbuttersäurehydrochlorid und Pyrazetam eingeführt. Als Kontrolle diente die physiologische Lösung (0,9%ige NaCl-Lösung). Die Untersuchungen wurden an I0 bis I2 erwachsenen Tieren (Mäuse, Ratten) durchgeführt.

Bei der Bestimmung der motorischen und Orientierungsaktivität wurde jede Maus in einer Einzelkamner des photoelektrischen Aktometers untergebracht. Die motorische und Orientierungsaktivität wurde nach der Zahl der Impulse bestimmt, die beim Berühren der Aktometerkontakte durch das Tier entstanden. Das Zählen der Im pulse wurde I0 min nach der intraperitonealen Einführung der genannten Verbindungen begonnen und dauerte innerhalb von 60 min. Die Angaben sind in der Tabelle I angeführt.

Tabelle I

| Verbindung, mg/kg | Dosis | Anzahl der Mäuse im Versuch | Anzahl der Impulse im Aktometer |
|---|---|---|---|
| Kontrolle: physiologische Lösung | | 24 | 379±36 |
| $\gamma$ -Amino- $\beta$ --phenylbuttersäurehydrochlorid | I6 | I2 | 368±5I |
| | 32 | I0 | 238±28 |
| | 40 | I0 | I58±2I |
| Erfindungsgemässe Verbindung | 25 | I0 | 322±43 |
| | 50 | I0 | 208±27 |
| | 75 | I2 | I23±I8 |
| Pyrazetam | I00 | I2 | 342±I8 |
| | 500 | I0 | 338±24 |
| | I000 | I0 | 288±28 |

Aus den in Tabelle angeführten Angaben ist ersichtlich, dass die beanspruchte Verbindung und $\gamma$ -Amino- $\beta$ -phenylbuttersäurehydrochlorid der Stärke der Hemmwirkung nach ungefähr gleich sind und dass der sedative Effekt von Pyrazetam bedeutend schwächer ist.

Die motorische Aktivität wurde in den Versuchen an Mäusen nach dem Test der rotierenden Stange bestimmt. Die motorische Fähigkeit wurde nach der Dauer des Festhaltens des Tiers an der Stange 75 min nach der Einführung der erwähnten Verbindungen eingeschätzt.

Die Angaben sind in der Tabelle 2 angeführt.

Tabelle 2

| Verbindung, mg/kg | Dosis | Anzahl der Mäuse im Versuch | Dauer des Festhaltens des Tiers an der rotierenden Stange, |
|---|---|---|---|
| Kontrolle: physiologische Lösung | | 25 | 85±II |
| γ-Amino-β-phenylbuttersäurehydrochlorid | 25 | I2 | 89±I8 |
| | 50 | I2 | 67±I3 |
| | I00 | I2 | 43±I5 |
| Erfindungsgemässe Verbindung | 25 | I0 | 83±I8 |
| | 75 | I2 | 52±I2 |
| | I50 | I2 | 29±I6 |
| Pyrazetam | I000 | I0 | 83±I9 |

Aus den in Tabelle 2 angeführten Angaben ist ersichtlich, dass die motorische Fähigkeit durch die neue beanspruchte Verbindung stärker als durch die bekannte Verbindung gehemmt wird.

Es wurde auch die emotionelle Aktivität und Aggressivität der Tiere auf dem Wege der elektrischen Schmerzreizung untersucht.

In einer Kammer von 20 x 20 x 25 cm Ausmass, deren Boden - ein Metallgitter aus Stangen darstellt, wurde ein Paar Tiere untergebracht (männliche Mäuse) und es wurde der Wechselstrom durchgelassen. Die Spannung wurde dosenartig auf 5 V solange erhöht, bis der Strom das Piepsen (die Schwelle der emotionellen Reaktivität) und die Schlägerei der Tiere (die Schwelle der Aggressivität) auslöste. Jede Spannungsgrösse wurde innerhalb von 5 sec zweimal in einem Zeitabstand von 20 bis 50 Sekunden aufrechterhalten. Die Kontrolle des Benehmens der Tiere begann man I0 Minuten nach der Einführung der oben erwähnten Verbindungen. Die Angaben sind in Tabellen 3, 4 angeführt.

Tabelle 3

| Verbindung, | Dosis mg/kg | Anzahl der Mäuse | Spannung in V | |
|---|---|---|---|---|
| | | | Schwelle der emotionellen Reaktivität | Schwelle der Agressivität |
| Kontrolle: physiologische Lösung | | 24 | 20,5±I,I | 28,4±I,4 |
| γ-Amino-β-phenylbuttersäurehydrochlorid | 25 | I0 | I9,9±I,8 | 26,7±I,3 |
| | 50 | I0 | 22,I±I,8 | 30,2±I,9 |
| | I00 | I0 | 24,2±0,8 | 3I,2±I,8 |
| Erfindungsgemässe Verbindung | 37 | 8 | 2I,8±I,6 | 29,8±2,4 |
| | 75 | I0 | 25,I±I,7 | 30,8±2,I |
| | I50 | I0 | 27,6±2,I | 33,6±I,8 |
| Pyrazetam | I00 | I0 | 2I,5±I,8 | 28,4±2,4 |
| | 500 | I0 | 20,4±I,0 | 29,8±I,8 |
| | I000 | I0 | 20,7±2,4 | 26,7±2,5 |

Tabelle 4

| Verbindung, | Dosis mg/kg | Anzahl der Ratten in der Gruppe | Spannung in V | |
|---|---|---|---|---|
| | | | die Schwelle der emotionellen Reaktivität | die Schwelle der Aggressivität |
| Kontrolle: physiologische Lösung | | I6 | 24,5±I,2 | 42,7±0,7 |
| γ-Amino-β-phenylbuttersäurehydrochlorid | 20,0 | 8 | 2I,7±I,6 | 33,4±I,9 |
| | 40,0 | 8 | 26,8±I,3 | 43,2±I,7 |
| | 80,0 | 8 | 33,2±I,6 | 44,6±0,6 |
| Erfindungsgemässe Verbindung | 20,0 | I0 | 23,4±0,8 | 37,8±I,7 |
| | 40,0 | I0 | 27,6±0,4 | 4I,2±I,3 |
| | 60,0 | I0 | 32,7±I,3 | 44,8±0,6 |
| | I50,0 | I0 | 34,2±2,2 | 45,2±I,6 |
| Pyrazetam | I00,0 | 8 | 25,7±0,8 | 40,6±I,4 |
| | 500,0 | 8 | 26,3±I,6 | 4I,8±I,2 |
| | I000,0 | 8 | 24,4±I,4 | 40,2±I,0 |

Aus den angeführten Angaben folgt, dass die erfindungsgemässe Verbindung eine deutlicher ausgeprägte tranguilizierende Wirkung auf die emotionelle Reaktivität und Aggressivität im Vergleich zu den bekannten angeführten Verbindungen besitzt.

Es wurde auch die Wirkung der erfindungsgemässen Verbindung unter Bedingungen einer Konfliktsituation an männlichen Mäusen (Gruppe aus I0 Mäusen) untersucht. Vorläufig wurde den Mäusen das Wasser für 48 Stunden entzogen, danach wurden ihnen 45 bis I20 Minuten vor dem Versuch die Verbindungen intraperitoneal eingeführt und die Tiere wurden in der Kammer untergebracht, wo sich eine Wassertränke befand. Im Augenblick der Wassereinnahme bekam es einen elektrischen Schlag, der am Zähler registriert wurde. In der Prüfkammer befand sich das Tier innerhalb von 3 Minuten. Für diese Zeit wurden die Zahl der Wassereinnahmen sowie die Lazenzperiode - die Zeit zwischen dem Unterbringen des Tiers in der Kammer und der ersten Wassereinnahme - registriert. Die durchschnittlichen Angaben sind in der Tabelle 5 angeführt.

Tabelle 5

| Verbindung, Dosis mg/kg | | Latenzperiode, sec | Anzahl der Wassereinnahmen für 3 Minuten |
|---|---|---|---|
| Kontrolle: physiologische Lösung | | 54,I±I9,2 | 2,7±0,9 |
| γ-Amino-β-phenylbuttersäurehydrochlorid | 4 | 32,4±I0,I | 8,5±I,8 |
| | 8 | 4I,6±7,5 | 7,8±I,4 |
| | I6 | 36,4±6,8 | 6,3±I,5 |
| | 32 | 49,2±I2,4 | 2,7±I,0 |
| Erfindungsgemässe Verbindung | 6,25 | 54,6±2I | 4,9±I,4 |
| | I2,5 | 48,2±I4 | 5,4±I,3 |
| | 25 | 52,4±20 | 5,6±0,7 |
| | 50 | 29,2±2I,0 | 4,4±I,2 |

Somit besitzt die erfindungsgemässe Verbindung eine mässige tranquilizierende Wirkung.

Ausserdem wurde die krampflösende Wirkung der neuen Verbindung untersucht. Die Untersuchung wurde an männlichen weissen Mäusen bei der Einführung von Pikrotoxin, Bicucullin, Korazol, Thiosemikarbazid durchgeführt.

Die beanspruchte Verbindung verlängert sicher die Krampflatenzzeit, aber verringert nicht den Anteil von Tieren, bei denen der Krampf beobachtet wurde.

γ -Amino- β -phenylbuttersäureorotat besitzt in Dosen von 50 bis 200 mg/kg keine ausgeprägte krampflösende Wirksamkeit.

Eis wurde die Wirkung der beanspruchten Verbindung auf den durch Phenamin ausgelösten Effekt der Veränderung der Hypermotorik untersucht. Phenamin wurde den Mäusen intraperitoneal in Dosen von 3 und 5 mg/kg I0 Minuten nach der enteralen oder intraperitonealen Einführung der beanspruchten Verbindung und des Pyrazetams eingeführt. Die Tiere wurden in der Kammer des photoelektrischen Aktometers untergebracht. Die motorische Aktivität wurde nach der Zahl der Impulse innerhalb von 60 Minuten bestimmt. Die Angaben sind in der Tabelle 6 angeführt.

Tabelle 6

| Verbindung, Dosis mg/kg | Anzahl der Mäuse im Versuch | Anzahl der Impulse im Aktometer innerhalb von 60 min |
|---|---|---|
| Kontrolle: physiologische Lösung | I2 | 428±3I |
| Kontrolle: Phenamin, 5 | IO | I92I±I4I |
| Phenamin, 5 + erfindungsgemässe Verbindung 50 | 8 | I884±III |
| Phenamin, 5 + erfindungsgemässe Verbindung 75 | IO | II25±I4I |
| Phenamin, 5 + erfindungsgemässe Verbindung IOO | 8 | 8I2±62 |
| Phenamin, 5 + erfindungsgemässe Verbindung I50 | IO | 8II±98 |
| Phenamin, 5 + Pyrazetam 500 | I2 | I872±66 |

Aus den in Tabelle angeführten Ergebnissen folgt, dass die beanspruchte Verbindung Substanz im Vergleich zu Pyrazetam eine ausgeprägte antagonistische Wirkung auf den psychostimulierenden Effekt von Phenamin ausübt.

Zur Untersuchung der Gewöhnung an die Wirkung der erfindungsgemässen Verbindung wurde die Dynamik der Benehmungseffekte an Mäusen beiderlei Geschlechts untersucht. In jeder Gruppe waren je I2 bis I8 Tiere. Die erfindungsgemässe Verbindung und $\gamma$ -Amino- $\beta$ - -phenylbuttersäurehydrochlorid wurden intraperitoneal in Dosen von 75 mg/kg bzw. 48 mg/kg zweimal am Tag eingeführt. Es wurden die Körpermasse, der Einfluss auf die motorische Aktivität (im Aktometer) und auf die emotionelle Reaktivität aud dem Wege der elektrischen Schmerzreizung untersucht. Die Tiere wurden innerhalb von 15 bis 20 Tagen untersucht. Die Körpermasse veränderte sich wesentlich nicht. Der sedative Effekt der beanspruchten Verbindung war während der ganzen Versuchsperiode aufrechterhalten (Tabelle 7).

Tabelle 7

| Verbindung, Dosis mg/kg | Anzahl der Mäuse in der Gruppe | Anzahl der Impulse im Aktometer innerhalb von 45 min |
|---|---|---|
| der I. Tag (nach der ersten Einführung der Verbindungen) | | |
| Kontrolle: physiologische Lösung | I2 | 354±60 |
| γ-Amino-β-phenylbuttersäurehydrochlorid 48 | I2 | 75±I9 |
| Die erfindungsgemässe Verbindung 75 | I2 | I25±25 |
| der 5. Tag (nach der ersten Einführung der Verbindungen) | | |
| Kontrolle: physiologische Lösung | I2 | 343±36 |
| γ-Amino-β-phenylbuttersäurehydrochlorid 48 | I2 | 223±36 |
| Die erfindungsgemässe Verbindung 75 | I2 | I08±2I |
| der TO. Tag (nach der ersten Einführung der Verbindungen) | | |
| Kontrolle: physiologische Lösung | I2 | 3I4±3I |
| γ-Amino-β-phenylbuttersäurehydrochlorid 48 | I2 | 257±38 |
| Die erfindungsgemässe Verbindung 75 | I2 | I09±47 |
| der I5. Tag (nach der ersten Einführung der Verbindungen) | | |
| Kontrolle: physiologische Lösung | I2 | 287±39 |
| γ-Amino-β-phenylbuttersäurehydrochlorid 48 | I2 | 283±47 |
| Die erfindungsgemässe Verbindung 75 | I2 | I23±I7 |

Aus der Tabelle ist zu ersehen, dass die neue Verbindung sowohl bei der einmaligen Einführung (der erste Versuchstag) als auch am 5., I0. und I5. Tag eine Hemmwirkung auf die motorische Aktivität des Tiers ausübte, während bei der Einführung von $\gamma$ -Amino--$\beta$ -phenylbuttersäurehydrochlorid eine allmähliche Abschwächung der sedativen Wirkung nachgewiesen wurde.

Die tranquilizierende Wirkung der beanspruchten Verbindung auf die emotionelle Reaktivität bleibt während der ganzen Periode der Einführung der neuen Verbindung erhalten (siehe Tabelle 8).

Tabelle 8

| Verbindung, Dosis mg/kg | Spannung in V | |
|---|---|---|
| | die Schwelle der emotionellen Reaktivität | die Schwelle der Aggressivität |
| I | 2 | 3 |
| der I. Tag nach der ersten Einführung | | |
| Kontrolle: physiologische Lösung | I7±2 | 22±2 |
| $\gamma$ -Amino- $\beta$ -phenylbuttersäurehydrochlorid 48 | 24±2 | 29±2 |
| Die erfindungsgemässe Verbindung 75 | 22±2 | 30±3 |
| der 5. Tag nach der ersten Einführung | | |
| Kontrolle: physiologische Lösung | I6±2 | I9±2 |
| $\gamma$ -Amino- $\beta$ -phenylbuttersäurehydrochlorid 48 | 23±2 | 27±4 |
| Die erfindungsgemässe Verbindung 75 | 23±2 | 29±2 |
| der I0. Tag nach der ersten Einführung | | |
| Kontrolle: physiologische Lösung | I5±2 | 20±2 |
| $\gamma$ -Amino- $\beta$ -phenylbuttersäurehydrochlorid 48 | 20±2 | 26±3 |
| Die erfindungsgemässe Verbindung 75 | 24±3 | 29±2 |

Fortsetzung der Tabelle 8

| I | | 2 | 3 |
|---|---|---|---|
| der I5. Tag nach der ersten Einführung | | | |
| Kontrolle: physiologische Lösung | | I5±2 | 20±I |
| γ-Amino-β-phenylbuttersäurehydrochlorid | 48 | I8±I | 24±2 |
| Die erfindungsgemässe Verbindung | 75 | 22±2 | 34±3 |

Aus den in der Tabelle angeführten Ergebnissen ist ersichtlich, dass für die erfindungsgemässe Verbindung die Erhöhung der Schwellen der emotionellen Reaktivität und der Aggressivität innerhalb von I5 Versuchttage zuverlässig aufrechterhalten bleibt, während die tranquilizierende Wirkung des γ -Amino- β -phenylbuttersäurehydrochlorids nur innerhalb von den ersten 5 Versuchstagen nachgewiesen wird.

Das Ausbleiben der Gewöhnung an die beanspruchte Verbindung wurde auch in den Rattenversuchen festgestellt, die im Laufe von 6 Monaten zur Bestimmung der motorischen Aktivität der Ratten durchgeführt wurden.

Die Angaben sind in der Tabelle 9 angeführt.

Tabelle 9

| Verbindung, Dosis mg/kg | | Zahl der Impulse im Aktometer innerhalb von 30 min |
|---|---|---|
| I | | 2 |
| der I. Tag de nach der ersten Einführung | | |
| Kontrolle: physiologische Lösung | | I57±I7 |
| Die erfindungsgemässe Verbindung | 25 | I83±24 |
| | 75 | III±I3 |
| | 200 | I5±I6 |
| der 35. Tag nach der ersten Einführung | | |
| Kontrolle: physiologische Lösung | | I8I±2I |
| Die erfindungsgemässe Verbindung | 25 | I7I±I8 |
| | 75 | I29±I4 |
| | 200 | 64±I8 |
| der 70. Tag nach der ersten Einführung | | |
| Kontrolle: physiologische Lösung | | I4I±I5 |
| Die erfindungsgemässe Verbindung | 25 | I26±27 |
| | 75 | 8I±I3 |
| | 200 | 43±I5 |
| der 85. Tag nach der ersten Einführung | | |
| Kontrolle: physiologische Lösung | | I6I±2I |
| Die erfindungsgemässe Verbindung | 25 | I47±I9 |
| | 75 | I09±I7 |
| | 200 | 67±2I |
| der I07. Tag nach der ersten Einführung | | |
| Kontrolle: physiologische Lösung | | I52±I8 |
| Die erfindungsgemässe Verbindung | 25 | I38±20 |
| | 75 | I37±I4 |
| | 200 | 79±I7 |
| der I20. Tag nach der ersten Einführung | | |
| Kontrolle: physiologische Lösung | | I72±3I |
| Die erfindungsgemässe Verbindung | 25 | I45±23 |
| | 75 | II8±28 |
| | 200 | 7I±I3 |

Fortsetzung der Tabelle 9

| I | | 2 |
|---|---|---|
| der I38. Tag nach der ersten Einführung | | |
| Kontrolle: physiologische Lösung | | I64±24 |
| Die erfindungsgemässe Verbindung | 25 | I68±32 |
| | 75 | I69±3I |
| | 200 | I03±I3 |
| der I54. Tag nach der ersten Einführung | | |
| Kontrolle: physiologische Lösung | | I4I±2I |
| Die erfindungsgemässe Verbindung | 25 | I27±I7 |
| | 75 | I8I±3I |
| | 200 | 85±24 |
| der I80. Tag nach der ersten Einführung | | |
| Kontrolle: physiologische Lösung | | I30±2I |
| Die erfindungsgemässe Verbindung | 25 | I54±30 |
| | 75 | I48±I3 |
| | 200 | II7±I8 |

Die antihypoxische Wirksamkeit der beanspruchten Verbindung wurde an verschiedenen Hypoxiemodellen untersucht.

Nach einer der Methoden wurden die Mäuse in der Barokammer untergebracht, die für die Höhe von II000 m über dem Meeresspiegel gehoben wurde. Die Hebung wurde mit einer Geschwindigkeit von 50 m/s verwirklicht. Der atmosphärische Tiefdruck wurde mit Hilfe der elektrischen Vakuumpumpe geschaffen.

Die beanspruchte Verbindung, Natriumoxybutyrat und Pyrazetam wurden 60 min vor dem Versuch enteral eingeführt. Der Schutzeffekt wurde nach der Überlebensdauer eingeschätzt. Die Angaben sind in Tabelle I0 angeführt.

Die antihypoxische Wirkung der erfindungsgemässen Verbindung ist stärker als die von Pyrazetam und Natriumoxybutyrat.

Nach der zweiten Methode wurden die Mäuse in einem hermetisch verschlossenen Gefäss von 80 $cm^3$ Inhalt untergebracht, dabei wurde die Luft nicht abgesaugt, und es wurden keine $CO_2$-Sorptionsmittel zugegeben. Die Prüfverbindungen wurden I Stunde vor dem Beginn des Versuchs intraperitoneal eingeführt (siehe Tabelle II).

Tabelle IO

| Verbindung, Dosis mg/kg | | Anzahl der Mäuse im Versuch | Überlebensdauer in min |
|---|---|---|---|
| Kontrolle: physiologische Lösung | | 3I | 2,3±0,4 |
| Die erfindungsgemässe Verbindung | 50,0 | I2 | 6,2±I,4 |
| | I00,0 | I2 | 7,5±2,I |
| | 200,0 | I2 | I5,2±I,8 |
| Natriumoxybutyrat | I00,0 | I0 | 7,I±2,3 |
| | 200,0 | I0 | I0,I±I,9 |
| Pyrazetam | I00,0 | 8 | 3,4±0,6 |
| | 500,0 | I0 | 5,5±I,8 |
| | I000,0 | I0 | 7,2±I,4 |

Tabelle II

| Verbindung, Dosis mg/kg | | Anzahl der Mäuse im Versuch | Überlebensdauer in min |
|---|---|---|---|
| Kontrolle: physiologische Lösung | | I5 | 9,8±0,5 |
| Die erfindungsgemässe Verbindung | 50,0 | I0 | I0,8±I,2 |
| | I00,0 | I0 | I5,2±I,4 |
| | 200,0 | I0 | 27,6±2,6 |
| Pyrazetam | I00,0 | I0 | 9,6±I,2 |
| | 500,0 | I0 | II,8±2,I |
| | I000,0 | I0 | I3,6±I,8 |

Im Vergleich zu Pyrazetam besitzt die erfindungsgemässe Verbindung eine viel deutlicher ausgeprägte antihypoxische Wirksamkeit.

Die gemische Hypoxie wurde durch die intraperitoneale und subkutane Verabreichung von Natriumnitrit in einer Dosis von 200 mg/kg den Gruppen zu I0 Ratten I5 min nach der Einführung der Prüfverbindungen erzeugt. Die Angaben sind in der Tabelle I2 (bei der intraperitonealen Einführung von Natriumnitrit) und Tabelle I3 (bei der subkutanen Einführung von Natriumnitrit) angeführt.

Tabelle I2

| Verbindung, Dosis mg/kg | | Anzahl der Mäuse im Versuch | Überlebensdauer in min |
|---|---|---|---|
| Kontrolle: physiologische Lösung | | 34 | 29,9±I,4 |
| Die erfindungsgemässe Verbindung | 50,0 | IO | 33,8±I,2 |
| | I00,0 | I2 | 53,I±4,I |
| | 200,0 | I2 | 46,8±2,7 |
| Natriumoxybutyrat | I00,0 | IO | 28,4±2,8 |
| | 200,0 | IO | 43,0±2,3 |
| Pyrazetam | I00,0 | 8 | 26,7±2,2 |
| | 500,0 | IO | 31,2±3,6 |
| | I000,0 | IO | 38,8±4,2 |

Tabelle I3

| Verbindung, Dosis mg/kg | Überlebensdauer in sec |
|---|---|
| Kontrolle: physiologische Lösung | 978,8±86 |
| Die erfindungsgemässe Verbindung, 50 einmalige Einführung | I300,6±III |
| Die erfindungsgemässe Verbindung, 50 chronische Einführung innerhalb von I2 Tagen einmal pro Tag | I620,0±I4I |
| $\gamma$-Amino-$\beta$-phenylbuttersäurehydrochlorid, 35 einmalige Einführung | I4I4,3±56 |
| $\gamma$-Amino-$\beta$-phenylbuttersäurehydrochlorid, 35 chronische Einführung innerhalb von I2 Tagen einmal pro Tag | II53,9±I02 |

Tabelle I4

| Verbindung, Dosis mg/kg | Überlebensdauer in sec |
|---|---|
| Kontrolle: physiologische Lösung | 87I,3±74 |
| Die erfindungsgemässe Verbindung, 50 chronische Einführung innerhalb von I6 Tagen einmal pro Tag | I295,0±92 |
| Die erfindungsgemässe Verbindung, 35 einmalige Einführung | II34,0±86 |
| $\gamma$-Amino-$\beta$-phenylbuttersäurehydrochlorid, 35 chronische Einführung innerhalb von I6 Tag einmal pro Tag | I205,I±III |
| Pyrazetam, 500 einmalige Einführung | 984,2±7I |

Die beanspruchte Verbindung übt eine starke antihypoxische Wirkung, insbesondere bei deren chronischer Einführung im Laufe von I2 bis I6 Tagen aus.

Die hypotoxische Hypoxie wurde durch die subkutane Einführung von Natriumnitroprussid in einer Dosis von 20 mg/kg den Gruppen von Mäusen und Ratten zu I0 Stück in der Gruppe erzeugt. Natriumnitroprussid wurde 60 min nach der Einführung der Prüfverbindungen verabreicht. Die Angaben sind in Tabelle I5 (für Mäuse) und I6 (für Ratten) angeführt.

Tabelle I5

| Verbindung, Dosis mg/kg | Überlebensdauer in sec |
|---|---|
| Kontrolle: physiologische Lösung | 5I5,0±46 |
| Die erfindungsgemässe Verbindung, 50 einmalige Einführung | 740,3±68 |
| Die erfindungsgemässe Verbindung, 50 chronische Einführung innerhalb von IO Tagen, einmal täglich | I083,7±80 |
| γ-Amino-β-phenylbuttersäurehydrochlorid, 35 chronische Einführung innerhalb von IO Tagen, einmal täglich | 5I5,6±34 |

Tabelle I6

| Verbindung, Dosis mg/kg | Überlebensdauer in sec |
|---|---|
| Kontrolle: physiologische Lösung | I009,5±56 |
| Die erfindungsgemässe Verbindung, 50 einmalige Einführung | I247,0±94 |
| Die erfindungsgemässe Verbindung, 50 chronische Einführung innerhalb von IO Tagen, einmal pro Tag | II33,0±54 |

Fortsetzung der Tabelle I6

| I | 2 |
|---|---|
| γ-Amino-β-phenylbuttersäurehydrochlorid, 35 einmalige Einführung | I359,0±I00 |
| γ-Amino-β-phenylbuttersäurehydrochlorid, 35 chronische Einführung innerhalb von IO Tagen, einmal pro Tag | 876,6±90 |

Dadurch ist aus den Üntersuchungsergebnissen der Wirkung der neuen Verbindung bei der subkutanen Einführung von Natriumnitroprussid sowie bei deren Untersuchung an allen früher beschriebenen Hypoxiemodellen ersichtlich, dass die beanspruchte Verbindung eine ausgeprägte antihypoxische Wirkung aufweist, dabei wird dieser Effekt bei der chronischen Anwendung dieser Verbindung stärker.

Der Einfluss der beanspruchten Verbindung auf die physische Toleranz der Tiere wurde nach dem Schwimmtest der Mäuse und Ratten untersucht. Die Versuche wurden in einer Wanne von 0,6 $cm^2$ Fläche durchgeführt. Die Tiergruppe (8 bis l0 Mäuse oder Ratten) wurde ins Wasser gebracht und es wurde die Zeit der Schwimmfähigkeit bestimmt. Die neue Verbindung und $\gamma$ -Amino- $\beta$ - -phenylbuttersäurehydrochlorid wurden enteral und intraperitoneal l Stunde oder 3 Stunden vor dem Schwimmbeginn eingeführt. Die Untersuchung wurde sowohl nach der einmaligen als auch nach der chronischen (innerhalb von 20 Tagen einmal täglich) Einführung der genannten Verbindungen durchgeführt. Die Versuche wurden bei 3 Temperaturen angestellt: 2 °C (Tabelle l7), 25 °C (Tabelle l8) und 40 °C (Tabelle l9).

Tabelle I7

| Verbindung, Dosis mg/kg | Schwimmzeit in | |
|---|---|---|
| | Mäuse | Ratten |
| Kontrolle: physiologische Lösung | I49,0±I2 | 278,3±32 |
| Die erfindungsgemässe Verbindung, 30 chronische Einführung innerhalb von 20 Tagen | 23I,5±35 | 390,0±56 |
| Die erfindungsgemässe Verbindung, 50 einmalige Einführung | I52,5±I0 | 282,0±I5 |
| γ-Amino-β-phenyl-buttersäurehydrochlorid, 35 chronische Einführung innerhalb von 20 Tagen | I20,8±25 | 328,8±34 |
| γ-Amino-β-phenyl-buttersäurehydrochlorid, 35 einmalige Einführung | I58,0±30 | 304,3±29 |

Tabelle I8

| Verbindung, Dosis mg/kg | | Schwimmzeit in | |
|---|---|---|---|
| | | Mäuse | Ratten |
| Kontrolle: physiologische Lösung | | I6 | 7,8±I,2 |
| Die erfindungsgemässe Verbindung | 50,0 | I2 | 8,2±0,5 |
| | I00,0 | I2 | I0,8±0,7 |
| | 200,0 | I2 | 7,6±I,7 |
| γ-Amino-β-phenyl-buttersäurehydrochlorid | 50,0 | I0 | 9,4±I,4 |
| | I00,0 | I0 | II,3±0,6 |
| | 200,0 | I0 | 9,4±I,4 |

Tabelle I9

| Verbindung, Dosis mg/kg (Anzahl der Mäuse im Versuch = IO) | Schwimmzeit in min |
|---|---|
| Kontrolle: physiologische Lösung | 50±4,5 |
| γ-Amino-β-phenylbuttersäurehydrochlorid, 35 einmalige Einführung | 37,8±8 |
| Die erfindungsgemässe Verbindung, 50 einmalige Einführung | 57,6±5,6 |
| γ-Amino-β-phenylbuttersäurehydrochlorid, 35 chronische Einführung während 20 Tage | 58±7,2 |
| Die erfindungsgemässe Verbindung, 50 chronische Einführung während 20 Tage | I28,6±I5 |

Aus den in Tabellen angeführten Ergebnissen ist zu ersehen, dass die einmalige Einführung der neuen Verbindung die Schwimmdauer der Mäuse und Ratten wesentlich nicht beeinflusst. Nach der 20 Tage langen chronischen Einführung der neuen Verbindung wird die Schwimmdauer hingegen zuverlässig grösser - (Tabelle I7) um das I,5fache im kalten Wasser und um das 2,5fache bei einer Temperatur von 40° C (Tabelle I9) im Vergleich zu γ -Amino- β -phenylbuttersäurehydrochlorid. Auf diese Weise erhöht die chronische Einführung der neuen Verbindung wesentlich die physische Toleranz der Tiere. Ausserdem wurde auch die Toleranz der Tiere gegen den Kältestress bestimmt. Die Untersuchung wurde bei einer Temperatur von 2° C an Mäusen und Ratten durchgeführt, indem jedes Tier abgesondert in der Einzelkammer von I5 x 20 x 20 cm Ausmass untergebracht wurde. Es wurde die Rektaltemperatur vor der Einführung der neuen Verbindung, des γ -Amino- β - phenylbuttersäurehydrochlorids und der physiologischen Kontrollösung eine Stunde nach der Einführung bei Zimmertemperatur und nach I Stunde und 2 Stunden in der Kälte gemessen. Die Versuche wurden an einer Mäusengruppe (I0 Stück) (Tabelle 20) und an einer Rattengruppe (8 Stück) (Tabelle 2I) bei der einmaligen und chronischen Einführung innerhalb von 22 bzw. I6 Tagen einmal pro Tag angestellt.

Tabelle 20

| Verbindung, Dosis mg/kg | Rektaltemperatur | | | |
|---|---|---|---|---|
| | vor der Einführung der Verbindung | I Stunde nach der Einführung der Verbindung | Nach I Stunde in der Kälte | Nach 2 Stunden in der Kälte |
| Kontrolle: physiologische Lösung | 33,4±0,I | 33,4±0,I | 28,8±0,3 | 26,I±0,I |
| Die erfindungsgemässe Verbindung, 50 einmalige Einführung | 32,9±0,2 | 3I,I±0,I | 29,8±0,I | 24,4±0,I |
| Die erfindungsgemässe Verbindung, 50 chronische Einführung während 22 Tage | 32,I±0,2 | 32,0±0,I | 29,5±0,3 | 29,6±0,I |
| γ-Amino-β-phenylbuttersäurehydrochlorid, chlorid, 35 einmalige Einführung | 33,7±0,3 | 32,2±0,2 | 26,3±0,2 | 27,8±0,3 |
| γ-Amino-β-phenylbuttersäurehydrochlorid, 35 chronische Einführung während 22 Tage | 30,9±0,I | 28,8±0,2 | 27,0±0,I | 26,0±0,3 |

Tabelle 2I

| Verbindung, Dosis mg/kg | Rektaltemperatur | | |
|---|---|---|---|
| | vor der Einführung der Verbindung | nach I Stunde | nach I Stunde an der Kälte |
| Kontrolle: physiologische Lösung | 30,4±0,3 | 30,5±0,I | 25,4±0,3 |
| Die erfindungsgemässe Verbindung, 50 chronische Einführung während I6 Tage | 30,7±0,2 | 27,2±0,I | 27,0±0,3 |
| Die erfindungsgemässe Verbindung, 50 einmalige Einführung | 30,4±0,3 | 30,9±C,3 | 25,I±0,3 |
| γ-Amino-β-phenylbuttersäurehydrochlorid, 35 chlorid, 35 chronische Einführung während I6 Tage | 29,7±0,I | 30,4±0,3 | 23,2±0,2 |

Aus den in Tabellen angeführten Angaben ist zu ersehen, dass zum Unterschied von γ-Amino-β-phenylbuttersäurehydrochlorid die neue Verbindung bei der chronischen Einführung die Tiere vor der Herabsetzung der Körpertemperatur bei der niedrigen Umgebungstemperatur deutlich schützt.

Es wurde auch der Einfluss der erfindungsgemässen Verbindung auf die durch Athanol erzeugte Intoxikation untersucht. In den Versuchen an Mäusen (in jedem Versuch wurde eine Gruppe von I0 Tieren untersucht) wurde die Überlebensdauer der Tiere bestimmt. Den Tieren wurde die Verbindung chronisch innerhalb von I4 Tagen einmal pro Tag enteral eingeführt. Athanol wurde I Stunde nach der Einführung der neuen Verbindung verabreicht. Die Ergebnisse dieser Untersuchung sind in der Tabelle 2 dargestellt.

Tabelle 22

| Verbindung, Dosis mg/kg | Überlebensdauer in sec nach der Einführung von Äthanol |
|---|---|
| Kontrolle: Äthylalkohol II ml/kg | I99,6±I0 |
| Die erfindungsgemässe Verbindung, 50 chronische Einführung während I4 Tage + Äthylalkohol | 283,8±I6 |
| Die erfindungsgemässe Verbindung, 50 einmalige Einführung + Äthylalkohol | 2I3,6±8 |

Aus den in Tabelle angeführten Ergebnissen ist ersichtlich, dass die chronische Einführung der neuen Verbindung die Überlebensdauer der Tiere um 30% verlängert.

Ausserdem wurde die Wirkung der beanspruchten Verbindung auf die durch Natriumäthaminal erzeugte Intoxikation untersucht. Die Versuche wurden an männlichen Mäusen und Ratten in Gruppen zu 8 bis I0 Tieren angestellt. Natriumäthaminal wurde intraperitoneal in einner Dosis von I00 mg/kg I5 min nach der enteralen Verabreichung der beanspruchten Verbindung oder der Kontrollarzneimittel eingeführt. Es wurde die einmalige und chronische Einwirkung (während I2 bis I4 Tage einmal täglich) von der neuen Verbindung, γ -Amino- β - -phenylbuttersäurehydrochlorid und Pyrazetam untersucht. Es wurde die Dauer der Seitenlage - des Schlafs der Tiere bestimmt. Die Ergebnisse dieser Untersuchung sind in Tabelle 23 (für Mäuse) und 24 (für Ratten) angeführt.

Aus den in Tabellen abgeführten Ergebnissen ist zu ersehen, dass die neue Verbindung zum Unterschied von γ -Amino- β -phenylbuttersäurehydrochlorid und Pyrazeram bei der chronischen Einführung eine bedeutende Herabset zung der Dauer des durch Natriumäthaminal erzeugten Schlafs hervorruft, eine ausgeprägte antitoxische Wirkung bei der Intoxikation mit Natriumäthaminal ausübt.

Tabelle 23

| Verbindung, Dosis mg/kg | Anzahl der Tiere | Schlafdauer in min |
|---|---|---|
| Kontrolle: Natriumäthaminal, I00 | I0 | 2I8,5±30 (Letalausgang) |
| Die erfindungsgemässe Verbindung, 50 einmalige Einführung + Natriumäthaminal | I0 | 26I,3±2I |
| Die erfindungsgemässe Verbindung, 50 chronische Einführung während I2 Tage + Natriumäthaminal | 9 | I78,4±I4 |
| γ -Amino- β -phenylbuttersäurehydrochlorid, 35 einmalige Einführung + Natriumäthaminal | I0 | 274,3±32 |
| γ -Amino- β -phenylbuttersäurehydrochlorid, 35 chronische Einführung während I2 Tage + Natriumäthaminal | 8 | 390,0±37 |

Tabelle 24

| Verbindung, Dosis mg/kg | Anzahl der Tiere | Schlafdauer in min |
|---|---|---|
| Kontrolle: Natriumäthaminal, I00 | I0 | 243,6±I3 |
| Die erfindungsgemässe Verbindung, 25 einmalige Einführung + Natriumäthaminal | I0 | 3I4,8±22 |
| Die erfindungsgemässe Verbindung, 50 einmalige Einführung + Natriumäthaminal | 8 | 306,4±I6 |
| Die erfindungsgemässe Verbindung, 50 chronische Einführung während I4 Tage + Natriumäthaminal | 8 | I67,4±23 |
| γ-Amino-β-phenylbuttersäurehydrochlorid, 50 einmalige Einführung + Natriumäthaminal | I0 | 288,6±27 |
| γ-Amino-β-phenylbuttersäurehydrochlorid, 50 chronische Einführung während I4 Tage + Natriumäthaminal | 9 | 290,0±I7 |
| Pyrazetam, 500 einmalige Einführung + Natriumäthaminal | I0 | 204,6±2I |

Unter Berücksichtigung der Tatsache, dass einige Verbindungen, die ähnlich mit der erfindungsgemässen Verbindung eine sedative Wirkung besitzen, als symptomatische Mittel zur Behandlung von verschiedenen Arrhythmienarten angewendet werden können, wurde der Einfluss der erfindungsgemässen Verbindung auf die toxischen Strophantin-Effekte geprüft, die mit der Herzrhythmusstörung verbunden sind. Dazu wurde bei weissen Mäusen (die Gruppe aus I0 Tieren) die Zeit des Todeseintritts bei der intravenösen Einführung von Strophantin (0,0I%ige wässrige Lösung) im einzelnen und in Kombination mit der neuen Verbindung und mit γ-Amino--β-phenylbuttersäurehydrochlorid bestimmt. Die Ergebnisse dieser Versuche sind in der Tabelle 25 angeführt.

Tabelle 25

| Verbindung in Kombination mit Strophantin | Dosis, mg/kg | Zeit des Todeseintritts, min |
|---|---|---|
| Kontrolle: Strophantin | I,7 | 9,0 (6,5 6-II,5) |
| γ-Amino-β-phenylbuttersäurehydrochlorid + Strophantin (I,7 mg/kg) | I0 | I0,0 (7,5 - I2,5) |
| γ-Amino-β-phenylbuttersäurehydrochlorid + Strophantin (I,7 mg/kg) | 90 | I7,2 (I0,I - 24,3) |
| Die erfindungsgemässe Verbindung + Strophantin (I,7 mg/kg) | I5 | II,7 (9,5 - I3,9) |
| Die erfindungsgemässe Verbindung + Strophantin (I,7 mg/kg) | 50 | 22,3 (8,9 - 35,6) |

Aus den in der Tabelle angeführten Ergebnissen ist zu ersehen, dass die erfindungsgemässe Verbindung zum Unterschied von γ -Amino- β -phenylbuttersäurehydrochlorid schon in geringen und mässigen (50 mg/kg) Dosen die Zeit des Todeseintritts der Tiere um das 2,5fache verlängert.

Die antiarrhythmischen Eigenschaften der erfindungsgemässen Verbindung wurden auch am Modell der Kalziumchloridarrhythmie untersucht. Der Mäusengruppe (I0 Gruppe) wurde einmalig die Kalziumchloridlösung intravenös im einzelnen und in Kombination mit γ -Amino-β -phenylbuttersäurehydrochlorid eingeführt. Die Dosis der letzteren betrug 50 mg/kg. Es wurde $LD_{50}$ für Kalziumchlorid bestimmt. Es wurde festgestellt, dass bei der Einführung der neuen Verbindung die Toxizität von Kalziumchlorid von 2I0 mg/kg auf 240 mg/kg sinkt, während γ -Amino- β -phenylbuttersäurehydrochlorid keinen Einfluss auf $LD_{50}$ von Kalziumchlorid ausübt.

Die akute Toxizität der beanspruchten Verbindung wurde an Mäusen und Ratten (die Gruppe aus 9 bis I2 Tieren) bei der intravenösen, intraperitonealen Einführung der beanspruchten Verbindung sowie bei deren Einführung in den Magen bestimmt.

Es wurden der letale Ausgang, der Allgemeinzustand, die Vergiftungssymptome registriert. $LD_{50}$ wurde nach der Methode von Lichtfield und Wilcoxon bestimmt. $LD_{50}$ der neuen Verbindung beträgt für Mäuse bei der intravenösen Einführung 580 mg/kg, bei der intraperitonealen Einführung I450 mg kg, bei der Einführung in den Magen 4600 mg/kg; für Ratten bei der intraperitonealen Einführung beträgt $LD_{50}$ 883 mg kg, bei der Einführung in den Magen - über I500 mg/kg.

Dadurch weist die erfindungsgemässe Verbindung eine niedrige Toxizität bei verschiedenen Einführungsverfahren auf.

Die erfindungsgemässe Verbindung besitzt somit eine ausgeprägte sedative, antihypoxische und antiarrhythmische Wirksamkeit. Sie ist wenig toxisch, übt keine reizende Wirkung auf die Schleimhaut des Verdauungskanals aus. Zum Unterschied von den bekannten Tranquillizern entwickelt sich bei der chronischen Einnahme der neuen Verbindung keine Gewöhnung. Die neue Verbindung erhöht die Toleranz gegen Hypothermie, Kältestress, physische Belastung, verbessert die motorische Fähigkeit, erhöht den Widerstand gegen die äthanolische Intoxikation,

Die Verbindung ist universell, weil es möglich ist, diese sowohl bei allen drei Indikationen zusammen als auch bei einer der Indikationen im einzelnen anzuwenden. Im letzteren Fall übt die neue Verbindung in bezug auf die anderen Indikationen keine Einwirkung aus. Nebenerscheinungen wurden nicht nachgewiesen.

Das Verfahren zur Herstellung von γ -Amino- β -. -phenylbuttersäurehydrochlorid ist technologisch einfach und wird wie folgt verwirklicht.

In den Reaktor werden Wasser, Orotsäure und γ -Amino- β -phenylbuttersäure eingetragen. Das

erhaltene Gemisch wird auf eine Temperatur von 96 bis 98 °C erhitzt. Das Gemisch wird bei dieser Temperatur für I Stunde stehengelassen. Aus dem erhaltenen Reaktionsgemisch wird das Endprodukt in an sich bekannter Weise isoliert.

Zum besseren Verständnis der vorliegenden Erfindung wird folgendes konkretes Beispiel angeführt.

Ausführungsbeispiel der Erfindung

Beispiel

In einen Reaktor werden 3000 ml Wasser, I56 g (I Mol) Orotsäure und 300 g (I,67 Mol) γ -Amino- β -phenylbuttersäure eingetragen. Das erhaltene Gemisch wird auf eine Temperatur von 96 bis 98 °C erwärmt und bei dieser Temperatur für I Stunde stehengelassen. Das erhaltene Reaktionsgemisch wird mit I0 g Aktivkohle bearbeitet, nachdem wird es abfiltriert. Das Filtrat wird innerhalb von 6 Stunden bei einer Temperatur von II bis I2 °C abgekühlt. Der ausgefallene Niederschlag wird abfiltriert und getrocknet. Man erhält 240 g γ -Amino- β -phenylbuttersäureorotat (72% der Theorie, bezogen auf Orotsäure).

Industrielle Verwertbarkeit

Die erfindungsgemässe Verbindung kann in der Medizin ihre Anwendung finden.

**Ansprüche**

γ -Amino- β -phenylbuttersäureorotat der allgemeinen Formel

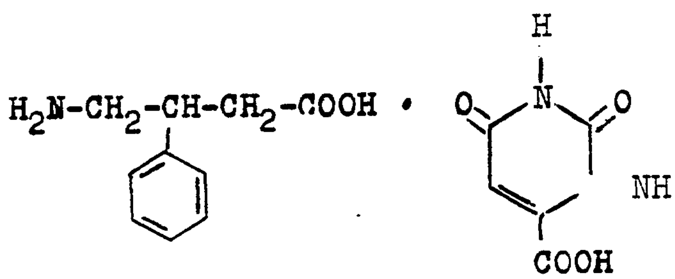

# INTERNATIONAL SEARCH REPORT

International Application No PCT/SU89/00063

**I. CLASSIFICATION OF SUBJECT MATTER** (if several classification symbols apply, indicate all) [6]

According to International Patent Classification (IPC) or to both National Classification and IPC

Int.Cl[5]: C07D 239/557, C07C 229/14, A61K 31/195

**II. FIELDS SEARCHED**

Minimum Documentation Searched [7]

| Classification System | Classification Symbols |
|---|---|
| Int.Cl.[4] | C07D 239/54, C07C 101/04, A61K 31/195 |

Documentation Searched other than Minimum Documentation to the Extent that such Documents are Included in the Fields Searched [8]

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | SU, AI, 198527 (A.G. Chigarev et al.) 28 July 1967 (28.07.67) | 1 |
| A | SU, AI, 236479 (Leningradsky gosudarstvenny pedagogichesky institut imeni A.I. Gertsena et al.) 24 November 1977 (24.11.77) | 1 |
| A | SU, AI, 313432 (Vsesojuzny nauchno-issledovatelsky vitaminny institut) 21 March 1972 (21.03.72) | 1 |
| A | SU, AI, 382337 (Leningradsky gosudarstvenny pedagogichesky institut imeni A.I. Gertsena et al.) 5 April 1976 (05.04.76) | 1 |
| A | GB, B, 1385846 (Leningradsky Gosudarstvenny pedagogichesky institut imeni A.I. Gertsena), 5 March 1975 (05.03.75) | 1 |

./.

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 30 October 1989 (30.10.89) | 6 December 1989 (06.12.89) |
| **International Searching Authority** | **Signature of Authorized Officer** |
| ISA/SU | |

| III. DOCUMENTS CONSIDERED TO BE RELEVANT (CONTINUED FROM THE SECOND SHEET) | | |
|---|---|---|
| Category * | Citation of Document, with indication, where appropriate, of the relevant passages | Relevant to Claim No |
| A | DE, A1, 2649503 (KLOSA, JOSEF) 3 May 1978 (03.05.78) | 1 |
| A | SU, AI, 988814 (Filial vsesojuznogo nauchno-issledovatelskogo khimiko - farmatsevticheskogo instituta imeni S. Ordzhonikidze) 15 January 1983 (15.01.83) | 1 |